# EUROPEAN PATENT SPECIFICATION

(11) **EP 0 515 434 B1**
(45) Date of publication and mention of the grant of the patent: **15.04.1998**
(21) Application number: 91903465.2
(22) Date of filing: 30.01.1991
(51) Int. Cl.: A61K 38/27

(54) **METHOD FOR TREATING INTESTINAL DISEASES**
METHODE ZUR BEHANDLUNG INTESTINALER KRANKHEITEN
PROCEDE DE TRAITEMENT DE TROUBLES INTESTINAUX

(30) Priority: 13.02.1990 AU 8586/90
(43) Date of publication of application: 02.12.1992
(73) Proprietor: GROPEP PTY. LTD., Adelaide, S.A. 5000 (AU)
(72) Inventor: BALLARD, Francis John, Kensington, S.A. 5068 (AU); READ, Leanna Christine, Kensington, S.A. 5068 (AU)
(74) Representative: Mallalieu, Catherine Louise
(86) International application number: AU9100031
(87) International publication number: WO9112018

(56) References cited:
- EP-A- 0 308 386
- WO-A-89/05822
- WO-A-90/15142
- WO-A-92/03155
- PEDIATRICS vol. 72, no. 4, 1983, ILLINOIS,USA pages 481 - 490 ROSENTHAL ET AL 'GROWTH FAILURE AND INFLAMMATORY BOWEL DISEASE: APPROACH TO TREATMENT OF A COMPLICATED ADOLESCENT PROBLEM'
- GASTROENTEROLOGY vol. 92, no. 5.2, 1987, NEW YORK,USA page 1468 KIRSCHNER ET AL 'FAILURE OF SOMATOMEDIN-C (SM-C) TO INCREASE AFTER TREATMENT OF CHILDHOOD CROHN'S DISEASE DISTINGUISHES DISEASE-RELATED GROWTH FAILURE FROM HYPOPITUITARISM'
- GASTROENTEROLOGY vol. 91, no. 4, 1986, NEW YORK,USA pages 830 - 836 KIRSCHNER ET AL 'SOMATOMEDIN-C LEVELS IN GROWTH-IMPAIRED CHILDREN AND ADOLESCENTS WITH CHRONIC INFLAMMATORY BOWEL DISEASE'
- JOURNAL OF BIOLOGICAL CHEMISTRY vol. 261, no. 31, 1986, BALTIMORE,USA pages 14539 - 14544 P.KAY LUND ET AL 'SOMATOMEDIN-C/INSULIN-LIKE GROWTH FACTOR-1 AND INSULIN-LIKE GROWTH FACTOR-II MRNAS IN RAT FETAL AND ADULT TISSUES'
- PROCEEDINGS OF THE NATIONAL ACADEMY OF SCIENCES,USA vol. 86, 1989, WASHINGTON D.C.,USA pages 7451 - 7455 WERNER ET AL 'DEVELOPMENTAL REGULATION OF THE RAT INSULIN-LIKE GROWTH FACTOR 1 RECEPTOR GENE'

## Description

This invention relates to the use of mammalian insulin-like growth factor-I (IGF0I) and its peptide analogues. More particularly, the invention relates to the use of IGF-I and its peptide analogues to treat disorders in gut function, e.g. relating to the effects of intestinal diseases.

IGF-I is a small protein that has been shown to stimulate the growth of cells in culture. Animal growth is also stimulated in pituitary-deficient, normal and catabolic states. Kidney function is also improved. These studies have led to the interpretation that IGF-I may be usefully applied in humans:
(1) to treat growth hormone deficiencies;
(2) to suppress the loss of body protein in catabolic states following burns, infection or other trauma; and
(3) as a treatment for patients suffering from renal diseases.

A number of human diseases result either in the subject having a lesser amount of gut tissue than required for normal digestion or absorption, or alternatively a diseased but normal-length gut in which digestion or absorption is impaired. Examples of human diseases that fit these two categories include short-gut syndrome, chronic ulcerative gut diseases, inflammatory gut diseases such as colitis and Crohn's disease, and necrotising enterocolitis in infants.

IGF-I is not known in the prior art to increase the growth or function of the cell types that comprise the abdominal gut, viz the stomach, duodenum, jejunum, ileum, cecum and colon. It is these regions of the gut that are affected in the diseases described above. Gut tissues do contain receptors for IGF-I (for example see: M. Laburthe et al, Am. J. Physiol. 254, G457, 1988; D.J. Pillion et al., Am. J. Physiol. 257, E27, 1989; H. Werner et al, Proc. Natl. Acad. Sci. USA 86, 7451, 1989), and are known under certain conditions to synthesise IGF-I or IGF-I messenger RNA (for example see: P.K. Lund et al., J. Biol. Chem. 261, 14539, 1986; A.J. D'Ercole et al., Pediatr. Res. 20, 253, 1986; H.-A. Hansson et al., Histochemistry 89, 403, 1988; W.L. Lowe, Jr., J. Clin. Invest. 84, 619, 1989).

IGF-I has previously been administered to dwarf mice, hypophysectomized rats, diabetic rats, starved mice and rats, normal rats, mini poodles and normal human subjects. The levels of IGF-I have also been increased by insertion of an IGF-I-expressing transgene in mice. In most of these studies IGF-I treatment has led to an increase in body growth. It has been noted that an undesirable side effect of high doses of IGF-I is hypoglycemia. However, in none of the reported studies was there any indication of an IGF-I effect on the abdominal gut. For example in one investigation where over expression of IGF-I was produced by transgenesis and led to substantial body growth in transgenic mice, the fractional weight of the duodenum was the same as in IGF-deficient animals (R.R. Behringer et al., Endocrinology 127, 1033, 1990). In another study the administration of low doses of IGF-I to suckling rats did not alter the weights of gastrointestinal organs (G.P. Young et al., Digestion 46S2, 240, 1990). In particular, there exists no prior report on the increase of gut weight following IGF-I administration.

EP-A-0308386 relates to the use of IGF-I for improving the regeneration of transected peripheral nerves in mammals.

WO89/05822 relates to IGF-I peptide analogues in which at least the glutamic acid is absent at position 3 from the N-terminal of IGF-I. These analogues are disclosed as being useful to treat growth hormone deficiencies.

WO90/15142 relates to expression vectors which comprise a first DNA sequence and a second DNA sequence which may be IGF-I or an analogue thereof. Treatment of protein accumulation deficiencies in mammals with the resulting fusion peptides is mentioned.

WO92/03155 relates to the use of IGF-I in the treatment or prevention of malfunction or disease of the intestinal mucosa.

We have now found it possible to overcome or at least alleviate, one or more of the difficulties related to the prior art.

The first aspect of the present invention concerns the use of mammalian, preferably human IGF-I for the manufacture of a pharmaceutical or veterinary preparation for the treatment of animals, including humans, with disorders in gut function.

More particularly, according to a first aspect of the present invention, there is provided the use of a mammalian insulin-like growth factor-I (IGF-I) or a peptide analogue thereof for the manufacture of a pharmaceutical or veterinary preparation for the treatment of disorders in gut function.

By the term "disorders in gut function", as used herein, we mean disorders in one or more of the stomach, duodenum, jejunum plus ileum, and colon. Such disorders may relate to intestinal diseases and/or surgical treatments. Thus where reference is made herein in general terms to the treatment of intestinal diseases, it is to be understood that we include the treatment of non-diseased intestines, part of which have been surgically removed and where increased growth of the residual intestine may be advantageous.

The term "peptide analogue", as used herein, includes one or more of the peptide analogues referred to in applicant's International Patent Publications WO87/01038, and WO89/05822, or fusion proteins derived therefrom as described in International Patent Publication WO90/15142.

An effective amount of mammalian IGF-I or an analogue of IGF-I is defined as that needed to increase the weight of gut tissue by more than approximately 20% above the weight of gut tissue prior to treatment.

Surprisingly IGF-I and its peptide analogues have now been found by the applicants to increase the weight of the stomach, duodenum, jejunum plus ileum, and colon as well as the total gut weight. Of particular significance is the fact that these effects on growth have been found to occur in animals under a range of conditions, including
(a) in animals in which gut function had previously been markedly compromised by the surgical removal of a substantial portion of the jejunum plus ileum
(b) animals to which glucocorticoids have been administered in order to produce a catabolic state
(c) animals in which diabetes had been induced by the drug streptozotocin, a condition that led to gut growth so that the effects of IGF-I were above an already-stimulated growth state, and
(d) animals which had compromised kidney function.

Since glucocorticoids are administered to humans as a current treatment for the inflammation that characterizes several gut diseases, the present discovery that gut growth can be partially restored by IGF-I or its analogues even in the presence of glucocorticoids is both surprising and advantageous.

In a preferred aspect of the present invention a peptide analogue to mammalian, preferably human, IGF-I is administered. Preferably the peptide analogue is an analogue wherein from 1 to 5 amino acid residues are absent from the N-terminal of mammalian IGF-I. Preferably 3 amino acid residues are absent therefrom. Such peptide analogue has been designated des(1-3)IGF-I.

Alternatively the peptide analogue may be an analogue wherein at least the glutamic acid residue is absent at position 3 from the N-terminal of mammalian IGF-I, and optionally replaced by a different amino acid residue. Preferably the analogue is one wherein the glutamic acid residue is replaced by an arginine residue.

More preferably the peptide analogue has an N-terminal sequence selected from 
Val-Leu-Cys-
Arg-Leu-Cys-
Gly-Leu-Cys-
Gly-Thr-Leu-Cys-
Gly-Pro-Arg-Thr-Leu-Cys-
Gly-Pro-Gly-Arg-Leu-Cys-
Gly-Pro-Gly-Gly-Leu-Cys-
Gly-Pro-Gly-Thr-Leu-Cys-
Gly-Pro-Gln-Thr-Leu-Cys-
Gly-Pro-Lys-Thr-Leu-Cys-
Gly-Pro-Leu-Thr-Leu-Cys-
 with the Cys residue shown being that normally at position 6 from the N-terminal.

In a further alternative aspect, the peptide analogue is a fusion protein including
a first amino acid sequence including approximately the first 100 N-terminal amino acids of methionine porcine growth hormone, or a fragment thereof; and
a second amino acid sequence of mammalian insulin-like growth factor-1, or an analogue thereof, joined to the C-terminal of the first amino acid sequence.

Preferably the first amino acid sequence includes approximately the first 46, more preferably the first 11, N-terminal amino acids of

methionine porcine growth hormone, or fragment thereof. The IGF-I analogues include des(1-3)IGF-I, specified in the co-owned International Publication WO87/01038, and had been shown to increase the growth of cultured cells at lower dose rates than required for IGF-I. In the development of the present invention, des(1-3)IGF-I has surprisingly been shown to show this increased potency in vivo, in the treatment of disorders in gut function, since' lower concentrations of the analogue than of IGF-I itself produce comparable increases in the growth of gut tissue.

The IGF-I analogues also include a fusion protein MpGH(11)VN/R³IGF-I (abbreviated to LR³), specified as Example 13 in the co-owned International Publication WO90/15142, and had been shown to increase the growth of cultured cells at lower dose rates than required for IGF-I. In the present invention, LR³ has also surprisingly been shown to show increased potency in the treatment of disorders in gut function, since lower concentrations of the analogue than of IGF-I itself produce comparable increases in the growth of gut tissue.

Although the use in particular applies to the treatment of human subjects with IGF-I or an analogue of IGF-I, it can also be applied veterinarily to animals with intestinal diseases.

Accordingly, in a further preferred aspect, the IGF-I or peptide analogue thereof is used in combination with a pharmaceutically or veterinarily acceptable diluent, carrier or excipient therefor. Preferably the IGF-I or the analogue of IGF-I is used in dose rates of approximately 10 to 2000, preferably 100 to 1000 micrograms/kg body weight/day. Treatment may continue for a period of approximately 1 to 60 days, preferably approximately 5 to 30 days.

The dose rate, dose intervals and treatment period may be adjusted to the degree of intestinal disease and the route of administration. Caution should be taken that blood glucose is monitored so that hypoglycemia can be prevented.

The dose rates and intervals for the administration of des(1-3)IGF-I and related analogues may be set at levels proportionally adjusted to the relative potency of the analogue to that of IGF-I itself. For example the levels for des(1-3)IGF-I or LR³ will be proportionally less than for the full IGF-I peptide in accordance with the increased potency of des(1-3)IGF-I or LR³. Dose rates of 50 to 500 micrograms of des(1-3)IGF-I LR³/kg body weight/day are preferred.

The pharmaceutical or veterinary preparations may be prepared utilising conventional techniques.

The benefits and parameters of the present invention will now be more fully described with reference to the accompanying examples. It should be understood, however, that the following description is illustrative only and should not be taken in any way as a restriction of the generality of the foregoing description.

### EXAMPLE 1

### Effects of IGF-I and des(1-3)IGF-I on stomach growth in the growth hormone-deficient lit/lit mouse

At 6 weeks of age lit/lit mice were housed individually and weighed on a daily basis, and by 8 weeks of age it had been established that body growth had virtually ceased.

Mice were then randomised into 5 groups according to body weight and sex and were injected daily for 20 days with
(a) 120 µl of a sterile solution containing one part of HCl (10 mmol/l) and nine parts of potassium phosphate (50 mmol/l), NaCl (150 mmol/l) and 0.1% human serum albumin at pH 7.4 (diluent),
(b) 120 µl of diluent containing 3 µg IGF-I,
(c) 120 µl of diluent containing 3 µg des(1-3)IGF-I,
(d) 120 µl of diluent containing 30 µg IGF-I, or
(e) 120 µl of diluent containing 30 µg des(1-3)IGF-I.
Each dose was administered subcutaneously as two injections, one at 9-10 am, the other at 4-5 pm. The animals were weighed and their lengths measured at 7 day intervals.

On day 21 the animals were killed by an anaesthetic overdose and tissues removed for weighing. The body weights, animal lengths (including tail) and stomach weights are shown in the following Table 1. Values are mans ± standard errors with statistical significance from diluent-treated control animals shown as *P<0.05; **P<0.01. The numbers of animals are given in parentheses.

Since the initial body weights were 10 grams for each group, the daily dose rates were approximately equivalent to 300 µg and 3000 µg of each peptide per kg body weight.

Stomach weights, expressed as a percentage of the diluent group, were 105% and 123% with 300 µg and 3000 µg/kg body weight/day of IGF-I respectively, and 110% and 123% with 300 µg and 3000 µg/kg body weight/day of des(1-3)IGF-1 respectively.

### EXAMPLE 2

### Effects of IGF-I and des(1-3)IGF-I LR³ on gut weights in dexamethasone-treated rats

Male Hooded Wistar rats, weighing on average 152 g (range 138-164 g) and maintained in metabolism cages, had Alzet model 2001 osmotic pumps inserted subcutaneously within the scapular region under ether anaesthesia. One pump delivered dexamethasone at 20 micrograms/d and the other either
(a) 0.1M acetic acid as diluent;
(b) IGF-I at 111 µg/d;
(c) IGF-I at 278 µg/d;
(d) IGF-I at 695 µg/d;
(e) des(1-3)IGF-I at 44 µg/d;
(f) des(1-3)IGF-I at 111 µg/d;
(g) des(1-3)IGF-I at 278 µg/d;
(h) LR³ at 44 µg/d;
(i) LR³ at 111 µg/d;
(j) LR³ at 278 µg/d.

Animals were maintained in the metabolism cages for 7 days with daily measurements of body weight, food and nitrogen intake and nitrogen excretion. After this period the animals were killed by exsanguination under anaesthesia and the gastro-intestinal tract from stomach to colon was removed and separated into stomach, duodenum, jejunum plus ileum, cecum and colon. All regions were cleared of food or fecal contents and weighed.

The body weights and the weights of the different regions of the gastrointestinal tract are given as means ± standard errors in Table 2. Statistical significance from the diluent-treated group is shown as *P<0.05; **P<0.01; ***P<0.001. There were six animals in each group.

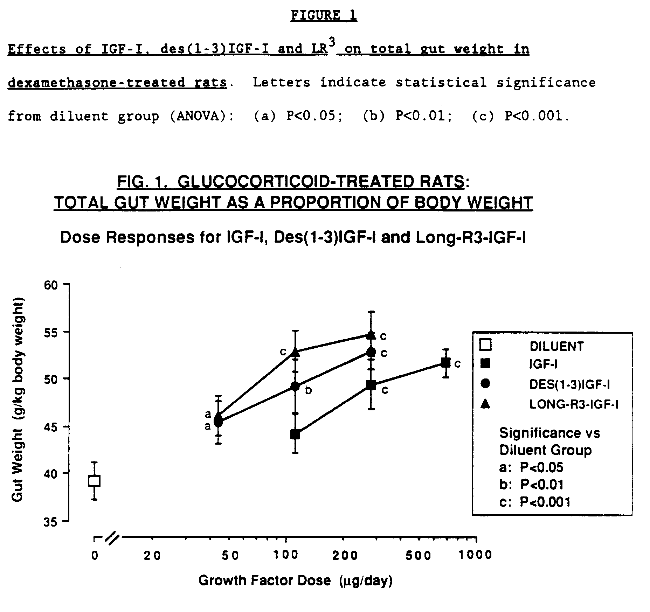

The weights of the total gut from stomach through colon is depicted in Figure 1 as a fraction of total body weight in the format of dose-response curves for IGF-I, des(1-3)IGF-I and LR³.

For a midpoint region of the ileum and the colon a portion was cut longitudinally and scraped to remove the mucosal layer. The weight of this layer was expressed as a percentage of the total mucosa plus muscularis. The protein contents of the same regions of the ileum were measured and expressed as mg per gram wet weight of tissue. These values are given as means ± standard errors in Table 3. Statistical significance from the diluent-treated animals was not achieved using ANOVA (P>0.05).

This example demonstrates marked increases in the weights of different regions of the gut of dexamethasone-treated rats following the administration OF IGF-I, des(1-3)IGF-I or LR³. The effects are dose dependent and are greater for des(1-3)IGF-I or LR³ at doses equivalent to IGF-I.

The increased growth occurs predominantly through an expansion in the cross-sectional area of the gut because the length of each region is either not increased or increased only slightly.

It is evident from Figure 1 that the increase in gut weight is proportionally above that occurring for body weight.

Both mucosal and muscularis regions of the jejunum and colon show increased growth because the percent by weight accounted for by the mucosa is not affected by IGF treatment.

Growth occurs by an increase in tissue protein since the percent protein content of the jejunum and colon is not changed by IGF treatment.

### EXAMPLE 3

### Quantitative histology of the duodenum from rats in Example 2

The mid region of the duodenum from certain animals in Example 1 was fixed in Bouin's fixative, dehydrated, embedded, transverse sections cut and stained with haematoxylin and eosin for quantitative histological analysis. All animals in groups (a), (c), (g) and (j) of Example 2 were processed and measurements of villus height, crypt depth, mucosal area, submucosal area, muscularis externa area and total cross-sectional area were obtained. For each duodenum, 30 villus heights, 30 crypt depths and 8 area measurements were averaged to obtain representative values. Means ± SEM (N=6) are shown in Table 4.

This example establishes that the growth of the duodenum produced by IGF-I, des(1-3)IGF-I or LR³ at dose rates of 278 µg/d is accompanied by statistically significant (P<0.01) increases in cross-sectional area. The increase is predominantly in the mucosal area, although the muscularis layer is also increased. The villi that make up much of the mocusa and play a key role in digestion and absorption, are also increased in height.

### EXAMPLE 4

### Effects of IGF-I and des(1-3)IGF-I on gut weights in rats treated following partial resection of the jejunum plus ileum

Male Sprague Dawley rats, weighing on average 175 g (range 160-193 g) and maintained in metabolism cages, had Alzet model 2001 osmotic pumps inserted as in Example 2. The dose rages of growth factors were 170 µg/d for IGF-I and for des(1-3)IGF-I as well as a higher dose rate of 425 µg IGF-I/d. At the same time as the pumps were inserted subcutaneously, and using tribromethanol in amylene hydrate anaesthesia and aseptic techniques, the jejunum plus ileum was exposed through a mid-line incision. The mid 80% of these parts of the intestine was removed starting 10 cm from the ligament of Treitz and finishing 10 cm from the ileo-cecal valve. The intestine and abdominal cavity were bathed frequently in sterile saline containing penicillin (1000 U/ml). To further guard against possible infection, the animals were injected with 0.6 ml of procaine penicillin before surgery and again 4 days later. The animals were returned to their metabolic cages and allowed free access to food and water. Body weights, food intakes, nitrogen intakes and nitrogen outputs were measured daily.

After 7 days of treatment, the animals were killed by exsanguination under anaesthesia and the gastro-intestinal tract from stomach to colon was removed and separated into stomach, duodenum, residual jejunum plus ileum, colon and cecum. All collected regions of the gut were cleared of food or fecal contents and weighed. The body weights and the weights of the different regions of the gut are given as means ± standard errors in Table 5. Statistical significance from the diluent-treated group is shown as *P<0.05; **P<0.01. There were 7 animals in the diluent and des(1-3)IGF-I groups, six in the group with the low dose of IGF-I and five in the group with the high dose of IGF-I.

The example shows that des(1-3)IGF-I and a 2.5 fold higher dose of IGF-I produce pronounced growth effects on the gut. A dose of IGF-I equal to that of des(1-3)IGF-I gave no statistically-significant effects.

The total gut weight less the residual jejunum and ileum, expressed as a percentage of the diluent group, was 113% with 170 µg IGF-I/d, 131% with 425 µg IGF-I/d, and 125% with 170 µg des(1-3)IGF-I/d.

### EXAMPLE 5

### Effects of IGF-I, des(1-3)IGF-I and MpGH(11)VN/R³IGF-I (LR³) on gut growth in diabetic rats

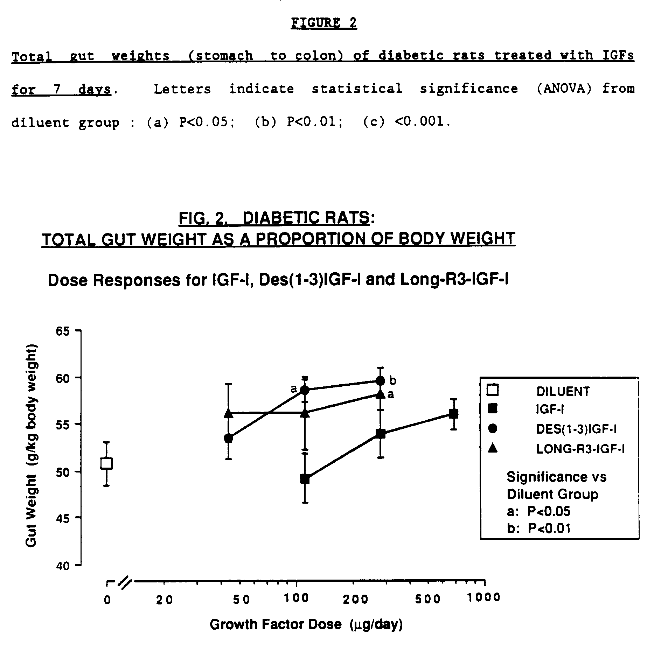

Male Hooded-Wistar rats weighing approximately 150 g were injected with streptozotocin intra-peritoneally at a dose of 70 mg/kg and transferred to metabolic cages. Diabetes was confirmed by blood glucose measurements. After 7 days (average body weight 162 g) the animals were implanted with osmotic pumps in exactly the same way and to deliver exactly the same doses as in Example 1. After 7 days treatment the animals were killed and the weights of the following gut organs measured: stomach, duodenum, ileum plus jejunum and colon. These values are shown in Table 6 and the total gut weights in Figure 2. Each value is the mean ± SEM for six animals. Statistical significance (ANOVA; least significant difference) is shown by *P<0.05, **P<0.01, ***P<0.001 versus diluent-treated diabetic rats.

These results demonstrate that IGF-I and lower doses of des(1-3)IGF-I or LR³ produce substantial growth effects on the stomach, ileum plus jejunum, colon and total gut weights. Effects on the duodenum are somewhat less.

The response of IGFs in the diabetic rats is especially important since even untreated animals already have heavier gut weights as a result of the hyperphagia associated with this condition. For example the weight of ileum plus jejunum was 5.08±0.29 g in another group of animals in which the diabetes was treated by insulin administration. This is lower than the diluent group in Table 5, notwithstanding the fact that the body weight of the insulin-treated animals was much heavier (231±8g).

### EXAMPLE 6

### Effects of IGF-I and des(1-3)IGF-I on gut weights in rats following partial nephrectomy

Partial renal failure was produced in Sprague Dawley male rats (95-125 g) by a 2-stage sub-total nephrectomy. This was performed via flank insicions, by ligating terminal branches of the left renal artery to give ischemia of at least half of the left kidney (Day 0), with a right nephrectomy being undertaken one week later, at which time the right jugular vein was also cannulated (Day 7). Partially nephrectomized rats were selected on day 14 for inclusion in the treatment period of the study on the basis of detectable proteinuria, and of increased urine volume and serum urea levels at least twoce those of the sham-operated control animals. On Day 16 treatment was commenced by means of mini-osmotic pumps (Alzet Model 2001, Alza Co., Palo Alto, California) which were implanted subcutaneously in the dorsal thoracic region under halothane anesthesia. Nephrectomized rats were randomly allocated to 4 treatment groups: diluent treated 0.1M acetic acid), low dose IGF-I treated (170 µg/d), high dose IGF-I treated (425 µg/d), or des(1-3)IGF-I treated (170 µg/d). The average body weight at time of pump insertion was 193 g. Animals were killed on Day 23 and gut weights measured as in Example 2. These are shown in Table 7. Statistical significance from diluent-treated animals is indicated by *P<0.05, **P<0.01.

This example demonstrates that gut weights are increased by 7 days treatment with IGF-I or des(1-3)IGF-I in rats that are compromised by partial nephrectomy.

**TABLE 1**

| Growth effects of IGF-I and des(1-3)IGF-I over a 20-day treatment period of lit/lit mice | | | | | |
|---|---|---|---|---|---|
| Treatment Group | | | Body Weight (g) | Animal Length (mm) | Stomach Weight (mg) |
| Diluent (8) | | | 10.20±0.19 | 124±2 | 64.3±3.0 |
| IGF-I, | 3 µg/d (8) | | 10.75±0.28 | 127±1 | 67.8±2.1 |
| | 30 µg/d (6) | | 11.25±0.41 | 132±1* | 79.0±3.0** |
| des(1-3)IGF-I, | | 3 µg/d (8) | 10.76±0.31 | 127±2 | 70.6±2.5 |
| | | 30 µg/d (6) | 11.33±0.42 | 132±1 | 79.3±4.4** |

| | | | | | |
|---|---|---|---|---|---|
| *P<0.05, | | | | | |
| **P<0.01, | | | | | |

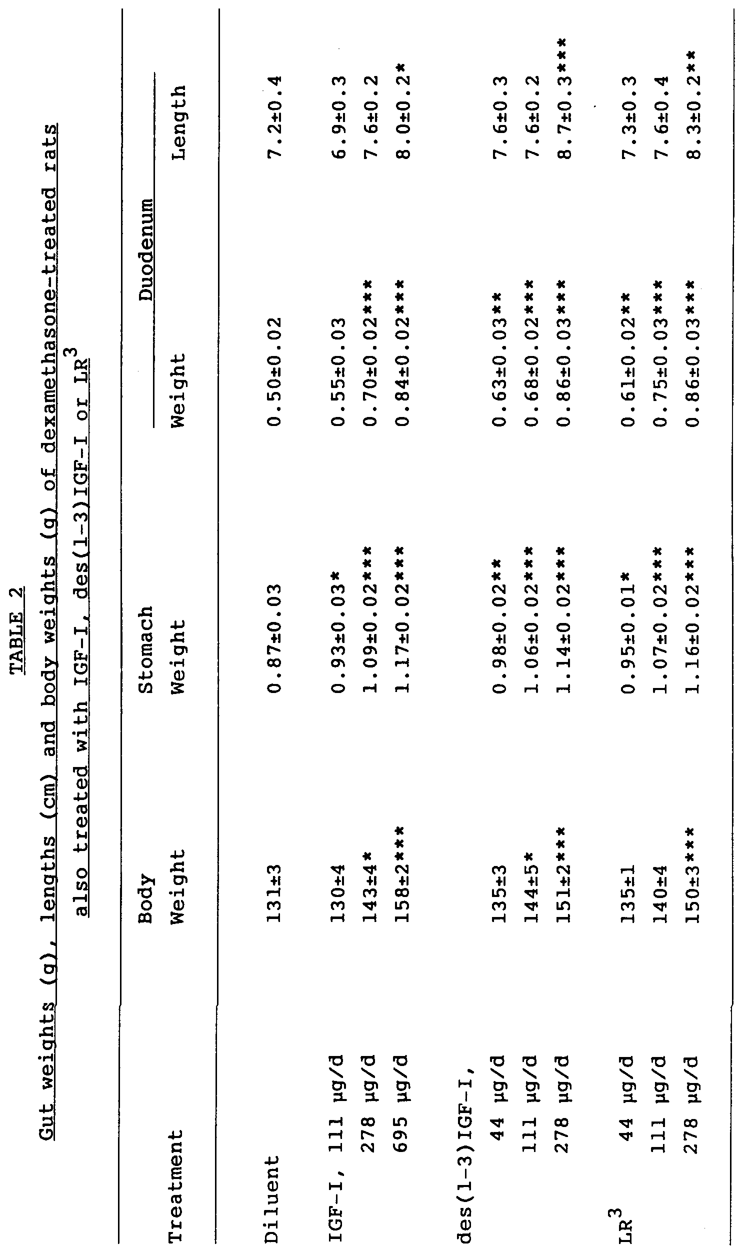

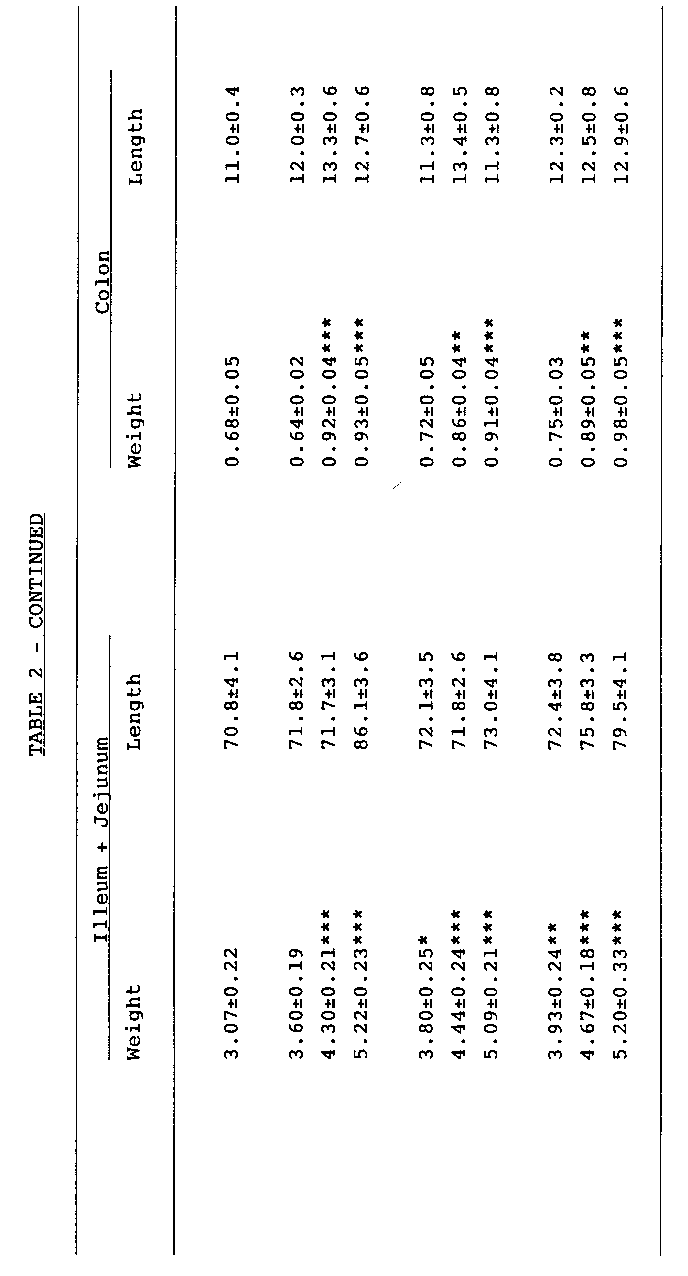

**TABLE 3**

| Fractional weights and protein contents of the jejunum in the mucosal layers in the jejunum and in the colon dexamethasone-treated rats treated with IGFs | | | | | | |
|---|---|---|---|---|---|---|
| Treatment | | | Jejunum | | | Colon Mucosa (% of total) |
| | | | Mucosa (% of total) | Mucosa Protein (mg/g weight) | Muscularis Protein (mg/g weight) | |
| Diluent | | | 55.1±3.3 | 124±5 | 169±4 | 30.5±2.5 |
| IGF-I, | 111 µg/d | | 58.8±2.2 | 117±6 | 151±4 | 29.6±1.7 |
| | 278 µg/d | | 62.7±1.5 | 135±5 | 161±5 | 31.7±1.7 |
| | 695 µg/d | | 61.1±2.8 | 136±5 | 159±2 | 28.8±1.2 |
| des(1-3)IGF-I, | | 44 µg/d | 53.3±3.9 | 134±5 | 161±5 | 31.1±1.7 |
| | | 111 µg/d | 59.6±2.3 | 140±5 | 162±4 | 27.5±2.2 |
| | | 278 µg/d | 55.3±3.6 | 140±4 | 154±6 | 32.5±2.5 |
| LR³, | 44 µg/d | | 56.8±4.2 | 119±10 | 147±4 | 30.2±2.8 |
| | 111 µg/d | | 60.7±3.3 | 139±4 | 168±6 | 29.6±2.1 |
| | 278 µg/d | | 54.2±3.2 | 135±6 | 159±5 | 30.6±2.2 |

**TABLE 4**

| Sections from rats treated with IGF-I des(1-3)IGF-I, LR³ or diluent | | | | |
|---|---|---|---|---|
| Measurement | Treatment Group | | | |
| | Diluent | IGF-I | des(1-3)IGF-I | LR³ |
| Villus height (mm) | 0.65±0.02 | 0.76±0.03 | 0.73±0.03 | 0.84±0.03*** |
| Crypt depth (mm) | 0.20±0.01 | 0.23±0.01 | 0.23±0.00 | 0.23±0.01 |
| Villus : crypt ratio | 3.3 ±0.2 | 3.4 ±0.2 | 3.3 ±0.1 | 3.7 ±0.2 |
| Mucosal area (mm²) | 5.67±0.44 | 7.72±0.56** | 8.03±0.58** | 8.29±0.56*** |
| Sub-mucosal area (mm²) | 0.42±0.04 | 0.51±0.04 | 0.52±0.03 | 0.51±0.04 |
| Musc. externa area (mm²) | 1.20±0.15 | 1.51±0.05* | 1.54±0.06** | 1.41±0.12 |
| Total area (mm²) | 7.29±0.61 | 9.74±0.59** | 10.10±0.62** | 10.21±0.66** |

| | | | | |
|---|---|---|---|---|
| *P<0.05, | | | | |
| **P<0.01, | | | | |
| ***P<0.001 versus diluent | | | | |

**TABLE 5**

| Duodenum weight (mg) | Diluent | IGF-I 170µg/d | IGF-I 425 µg/d | des(1-3)IGF-1 170 µg/d |
|---|---|---|---|---|
| Body weight (g) | 171.6 ± 4.5 | 173.4 ± 5.9 | 190.9 ± 4.2 | 184.1 ± 5.6 |
| Stomach weight (mg) | 980 ± 75 | 1089 ± 47 | 1236** ± 26 | 1136* ± 52 |
| Duodenum weight (mg) | 1067 ± 75 | 1179 ± 88 | 1572** ± 81 | 1542** ± 102 |
| Residual Jejunum + Ileum weight (mg) | 1984 ± 118 | 2217 ± 244 | 2288 ± 60 | 2259 ± 150 |
| Colon weight (g) | 1067 ± 162 | 1305 ± 164 | 1330 ± 65 | 1250 ± 127 |
| Total gut weight less residual jejunum & ileum (g) | 4.28 ± 0.27 | 4.83 ± 0.34 | 5.59** ± 0.13 | 5.37** ± 0.24 |

| | | | | |
|---|---|---|---|---|
| *P<0.05, | | | | |
| **P<0.01, | | | | |

**TABLE 6**

| Gut weights (g) of diabetic rats and body weights (g) | | | | | | |
|---|---|---|---|---|---|---|
| | | Body Weight | Stomach | Duodenum | Ileum + Jejunum | Colon |
| Diluent | | 172±6 | 1.01±0.04 | 0.72±0.09 | 6.10±0.49 | 0.95±0.02 |
| IGF-I, | 111 µg/d | 186±4 | 1.13±0.04 | 0.74±0.08 | 6.21±0.37 | 1.05±0.07 |
| | 278 µg/d | 198±8* | 1.19±0.06* | 0.91±0.05 | 7.41±0.32* | 1.09±0.05 |
| | 695 µg/d | 210±8*** | 1.44±0.08*** | 0.94±0.06* | 8.17±0.32*** | 1.18±0.05** |
| des(1-3)IGF-I, | 44 µg/d | 185±6 | 1.23±0.05** | 0.82±0.11 | 6.83±0.38 | 1.02±0.05 |
| | 111 µg/d | 194±7* | 1.27±0.06** | 0.89±0.07 | 7.97±0.20** | 1.16±0.08** |
| | 278 µg/d | 212±8*** | 1.49±0.07*** | 1.02±0.09** | 8.69±0.40*** | 1.41±0.03*** |
| LR³ | 44 µg/d | 190±7 | 1.14±0.06 | 0.85±0.07 | 7.58±0.48* | 1.05±0.04 |
| | 111 µg/d | 204±6** | 1.38±0.04*** | 0.88±0.11 | 7.83±0.53** | 1.25±0.07*** |
| | 278 µg/d | 213±8*** | 1.48±0.08*** | 0.91±0.06 | 8.47±0.46*** | 1.50±0.04*** |

| | | | | | | |
|---|---|---|---|---|---|---|
| *P<0.05, | | | | | | |
| **P<0.01, | | | | | | |
| ***P<0.001 versus diluent | | | | | | |

**TABLE 7**

| Gut weights (g) in partially-nephrectomised rats treated with IGF-I or des(1-3)IGF-I | | | | | | |
|---|---|---|---|---|---|---|
| Treatment | | Stomach | Duodenum | Ileum + Jejunum | Colon | Total |
| Diluent | | 1.08±0.04 | 0.51±0.02 | 3.78±0.19 | 0.83±0.03 | 6.21±0.22 |
| IGF-I, | 170 µg/d | 1.19±0.03 | 0.63±0.03* | 4.70±0.27** | 0.97±0.03* | 7.50±,0.30** |
| | 425 µg/d | 1.25±0.06* | 0.75±0.07** | 5.11±0.22** | 1.05±0.07** | 8.16±0.27** |
| des(1-3)IGF-I | 170 µg/d | 1.16±0.06 | 0.66±0.03* | 4.48±0.15* | 0.95±0.06 | 7.24±0.10** |

| | | | | | | |
|---|---|---|---|---|---|---|
| *P<0.05, | | | | | | |
| **P<0.01, | | | | | | |

## Claims

1. Use of a mammalian insulin-like growth factor-I (IGF-I) or a peptide analogue thereof for the manufacture of a pharmaceutical or veterinary preparation for the treatment of disorders in gut function.

2. Use according to Claim 1 wherein the IGF-I or peptide analogue thereof is for administration in an amount of from approximately 10 to 2000 microgram/kg body weight/day for a period of approximately 1 to 60 days.

3. Use according to Claim 1, the mammalian insulin-like growth factor-I is a human insulin-like growth factor-I.

4. Use according to claim 3, wherein 3 amino acid residues are absent from the N-terminal of the growth factor.

5. Use according to claim 1, wherein from 1 to 5 amino acid residues are absent from the N-terminal of the peptide analogue of mammalian insulin-like growth factor-I.

6. Use according to claim 5, wherein the peptide analogue is for administration at a dose rate of approximately 50 to 500 micrograms/kg body weight/day for a period of approximately 1 to 60 days.

7. Use according to claim 1, wherein the peptide analogue of the mammalian insulin-like growth factor-I has at least the glutamic acid residue absent at position 3 from the N-terminal thereof, and which is optionally replaced by a different amino acid residue.

8. Use according to claim 7, wherein the glutamic acid residue is replaced by an argenine residue.

9. Use according to claim 7, wherein the peptide analogue has an N-terminal sequence selected from 
Val-Leu-Cys-
Arg-Leu-Cys-
Gly-Leu-Cys-
Gly-Thr-Leu-Cys-
Gly-Pro-Arg-Thr-Leu-Cys-
Gly-Pro-Gly-Arg-Leu-Cys-
Gly-Pro-Gly-Gly-Leu-Cys-
Gly-Pro-Gly-Thr-Leu-Cys-
Gly-Pro-Gln-Thr-Leu-Cys-
Gly-Pro-Lys-Thr-Leu-Cys-
Gly-Pro-Leu-Thr-Leu-Cys-
 with the Cys residue shown being that normally at position 6 from the N-terminal.

10. Use according to claim 7 wherein the peptide analogue is for administration at a dose rate of approximately 50 to 500 micrograms/kg body weight/day for a period of approximately 1 to 4 days.

11. Use according to claim 1, wherein the peptide analogue of the mammalian insulin-like growth factor-I is a fusion protein including
a first amino acid sequence including approximately the first 100 N-terminal amino acids of methionine porcine growth hormone, or a fragment thereof; and
a second amino acid sequence of mammalian insulin-like growth factor-I, or an analogue thereof, joined to the C-terminal of the first amino acid sequence.

12. Use according to claim 11, wherein said first amino acid sequence includes approximately the first 46 N-terminal amino acids of methionine porcine growth hormone, or fragment thereof.

13. Use according to claim 12 wherein said first amino acid sequence includes approximately the first 11 N-terminal amino acids of methionine porcine growth hormone, or fragment thereof.

14. Use according to claim 11 wherein the peptide analogue is for administration at a dose rate of approximately 50 to 500 microgram/kg body weight/day for a period of approximately 1 to 60 days.

## Patentansprüche

1. Verwendung eines Säuger-insulinähnlichen-Wachstumsfaktors-I (IGF-I) oder eines Peptidanalogen davon, zur Herstellung eines Arzneimittels oder eines Tierarzneimittels zur Behandlung von Störungen der Darmfunktion.

2. Verwendung nach Anspruch 1, worin das IGF-I oder dessen Peptidanaloges zur Verabreichung in einer Menge von etwa 10 bis 2000 Mikrogramm/kg Körpergewicht/Tag über einen Zeitraum von etwa 1 bis 60 Tagen bestimmt ist.

3. Verwendung nach Anspruch 1, worin der Säuger-insulinähnliche-Wachstumsfaktor-I ein Human-insulinähnlicher-Wachstumsfaktor-I ist.

4. Verwendung nach Anspruch 3, worin 3 Aminosäurereste am N-Terminus des Wachstumsfaktors fehlen.

5. Verwendung nach Anspruch 1, worin 1 bis 5 Aminosäurereste am N-Terminus des Peptidanalogen des Säuger-insulinartigen-Wachstumsfaktors-I fehlen.

6. Verwendung nach Anspruch 5, worin das Peptidanaloge zur Verabreichung mit einer Dosisrate von etwa 50 bis 500 Mikrogramm/kg Körpergewicht/Tag über einen Zeitraum von etwa 1 bis 60 Tagen bestimmt ist.

7. Verwendung nach Anspruch 1, worin im Peptidanalogen des Säuger-insulinähnlichen-Wachstumsfaktors-I mindestens der Glutaminsäurerest in Position 3 zum N-Terminus fehlt und dieser gegebenenfalls durch einen anderen Aminosäurerest ersetzt ist.

8. Verwendung nach Anspruch 7, worin der Glutaminsäurerest durch einen Argininrest ersetzt ist.

9. Verwendung nach Anspruch 7, worin das Peptidanaloge eine N-Terminus-Sequenz, ausgewählt aus 
Val-Leu-Cys-
Arg-Leu-Cys-
Gly-Leu-Cys-
Gly-Thr-Leu-Cys-
Gly-Pro-Arg-Thr-Leu-Cys-
Gly-Pro-Gly-Arg-Leu-Cys-
Gly-Pro-Gly-Gly-Leu-Cys-
Gly-Pro-Gly-Thr-Leu-Cys-
Gly-Pro-Gln-Thr-Leu-Cys-
Gly-Pro-Lys-Thr-Leu-Cys-
Gly-Pro-Leu-Thr-Leu-Cys-
 hat, wobei der dargestellte Cys-Rest derjenige ist, der sich normalerweise in der Position 6 zum N-Terminus befindet.

10. Verwendung nach Anspruch 7, worin das Peptidanaloge zur Verabreichung mit einer Dosisrate von etwa 5 bis 500 Mikrogramm/kg Körpergewicht/Tag über einen Zeitraum von etwa 1 bis 4 Tagen bestimmt ist.

11. Verwendung nach Anspruch 1, worin das Peptidanaloge des Säuger-insulinähnlichen-Wachstumsfaktors-I ein Fusionsprotein darstellt, welches enthält:
eine erste Aminosäuresequenz, welche etwa die ersten 100 N-terminalen Aminosäuren des Methionin-Schweinewachstumshormons oder ein Fragment davon enthält; und
eine zweite Aminosäuresequenz aus Säuger-insulinähnlichem-Wachstumsfaktor-I oder einem Analogen davon, das an den C-Terminus der ersten Aminosäuresequenz gebunden ist.

12. Verwendung nach Anspruch 11, worin die erste Aminosäuresequenz ungefähr die ersten 46 N-terminalen Aminosäuren des Methionin-Schweinewachstumshormons oder eines Fragments davon enthält.

13. Verwendung nach Anspruch 12, worin die erste Aminosäuresequenz ungefähr die ersten 11 N-terminalen Aminosäuren des Methionin-Schweinewachstumshormons oder eines Fragments davon enthält.

14. Verwendung nach Anspruch 11, worin das Peptidanaloge zur Verabreichung mit einer Dosisrate von etwa 50 bis 500 Mikrogramm/kg Körpergewicht/Tag über einen Zeitraum von etwa 1 bis 60 Tagen bestimmt ist.

## Revendications

1. Utilisation d'un facteur de croissance analogue à l'insuline de type I (IGF-I) mammifère ou de l'un de ses analogues peptidiques pour la fabrication d'une préparation pharmaceutique ou vétérinaire en vue du traitement de désordres dans le fonctionnement intestinal.

2. Utilisation suivant la revendication 1, dans laquelle l'IGF-I ou l'un de ses analogues peptidiques convient à une administration selon une quantité d'environ 10 à 2000 µg/kg de poids corporel/j pendant une période d'environ 1 à 60 jours.

3. Utilisation suivant la revendication 1, dans laquelle le facteur de croissance analogue à l'insuline de type I mammifère est un facteur de croissance analogue à l'insuline de type I humain.

4. Utilisation suivant la revendication 3, dans laquelle 3 restes aminoacides sont absents à partir de l'extrémité N-terminale du facteur de croissance.

5. Utilisation suivant la revendication 1, dans laquelle 1 à 5 restes aminoacides sont absents à partir de l'extrémité N-terminale de l'analogue peptidique du facteur de croissance analogue à l'insuline de type I mammifère.

6. Utilisation suivant la revendication 5, dans laquelle l'analogue peptidique convient à une administration selon une dose d'environ 50 à 500 µg/kg de poids corporel/j pendant une période d'environ 1 à 60 jours.

7. Utilisation suivant la revendication 1, dans laquelle l'analogue peptidique du facteur de croissance analogue à l'insuline de type I mammifère a au moins le reste acide glutamique absent en position 3 de son extrémité N-terminale, ledit reste étant le cas échéant remplacé par un reste aminoacide différent.

8. Utilisation suivant la revendication 7, dans laquelle le reste acide glutamique est remplacé par un reste arginine.

9. Utilisation suivant la revendication 7, dans laquelle l'analogue peptidique a une séquence N-terminale choisie parmi les : Val-Leu-Cys- 
Arg-Leu-Cys-
Gly-Leu-Cys-
Gly-Thr-Leu-Cys-
Gly-Pro-Arg-Thr-Leu-Cys-
Gly-Pro-Gly-Arg-Leu-Cys-
Gly-Pro-Gly-Gly-Leu-Cys-
Gly-Pro-Gly-Thr-Leu-Cys-
Gly-Pro-Gln-Thr-Leu-Cys-
Gly-Pro-Lys-Thr-Leu-Cys-
Gly-Pro-Leu-Thr-Leu-Cys-
 où le reste Cys tel que représenté est celui qui est normalement en position 6 de l'extrémité N-terminale.

10. Utilisation suivant la revendication 7, dans laquelle l'analogue peptidique convient à une administration selon une dose d'environ 50 à 500 µg/kg de poids corporel/j pendant une période d'environ 1 à 4 jours.

11. Utilisation suivant la revendication 1, dans laquelle l'analogue peptidique du facteur de croissance analogue à l'insuline de type I mammifère est une protéine de fusion comprenant :
une première séquence aminoacide contenant approximativement les 100 premiers aminoacides N-terminaux de la méthionine-hormone de croissance porcine ou l'un des ses fragments ; et
une seconde séquence aminoacide du facteur de croissance analogue à l'insuline de type I mammifère, ou l'un de ses analogues, qui est jointe à l'extrémité C-terminale de la première séquence aminoacide.

12. Utilisation suivant la revendication 11, dans laquelle ladite première séquence aminoacide comporte approximativement les 46 premiers aminoacides N-terminaux de la méthionine-hormone de croissance porcine ou l'un des ses fragments.

13. Utilisation suivant la revendication 12, dans laquelle ladite première séquence aminoacide comporte approximativement les 11 premiers aminoacides N-terminaux de la méthionine-hormone de croissance porcine ou l'un des ses fragments.

14. Utilisation suivant la revendication 11, dans laquelle l'analogue peptidique convient à une administration selon une dose d'environ 50 à 500 µg/kg de poids corporel/j pendant une période d'environ 1 à 60 jours.
